(19) Europäisches Patentamt
European Patent Office
Office européen des brevets

(11) EP 0 750 672 B1

(12) **EUROPÄISCHE PATENTSCHRIFT**

(45) Veröffentlichungstag und Bekanntmachung des Hinweises auf die Patenterteilung:
**28.11.2001 Patentblatt 2001/48**

(21) Anmeldenummer: **95915743.9**

(22) Anmeldetag: **17.03.1995**

(51) Int Cl.[7]: **C12N 15/57**, C12N 9/64, G01N 33/50, C12N 1/21, C12N 1/19, C12N 5/10

(86) Internationale Anmeldenummer:
**PCT/DE95/00357**

(87) Internationale Veröffentlichungsnummer:
**WO 95/25171 (21.09.1995 Gazette 1995/40)**

(54) **DNA-SEQUENZEN FÜR MATRIX-METALLPROTEASEN, IHRE HERSTELLUNG UND VERWENDUNG**

DNA SEQUENCES FOR MATRIX METALLOPROTEASES, THEIR PRODUCTION AND USE

SEQUENCES D'ADN POUR METALLOPROTEASES MATRICIELLES, LEUR PRODUCTION ET LEUR UTILISATION

(84) Benannte Vertragsstaaten:
**AT BE CH DE DK ES FR GB GR IE IT LI LU MC NL PT SE**

(30) Priorität: **17.03.1994 DE 4409663**
**21.10.1994 DE 4438838**

(43) Veröffentlichungstag der Anmeldung:
**02.01.1997 Patentblatt 1997/01**

(73) Patentinhaber: **MAX-DELBRÜCK-CENTRUM FÜR MOLEKULARE MEDIZIN**
**13125 Berlin (DE)**

(72) Erfinder:
- **WILL, Horst**
  **13125 Berlin (DE)**
- **HINZMANN, Bernd**
  **13127 Berlin (DE)**

(74) Vertreter: **Jacobi, Markus Alexander**
**Hoechst Marion Roussel Deutschland GmbH,**
**Patent- und Lizenzabteilung,**
**Geb. K 801**
**65926 Frankfurt am Main (DE)**

(56) Entgegenhaltungen:
**EP-A- 0 685 557**

- **NATURE, Bd. 370, 7.Juli 1994 LONDON GB, Seiten 61-65, HIROSHI SATO ET AL. 'A matrix metalloproteinase expressed on the surface of invasive tumour cells'**
- **EUROPEAN JOURNAL OF BIOCHEMISTRY, Bd. 231, Nr. 3, August 1995 Seiten 602-608, WILL H. ET AL. 'cDNA sequence and mRNA tissue distribution of a novel human matrix metalloproteinase with a potential transmembrane segment'**
- **NATURE, Bd. 348, Dezember 1990 LONDON GB, Seiten 699-704, BASSET P. ET AL. 'A novel metalloproteinase gene specifically expressed in stromal cells of breast carcinomas'**
- **BIOESSAYS, Bd. 14, Nr. 7, Juli 1992 Seiten 455-463, MATRISIAN L. M. 'The matrix-degrading metalloproteinases' in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft DNA-Sequenzen für menschliche Matrix-Metalloproteasen sowie davon abgeleitete und homologe Sequenzen. Sie betrifft ferner die durch die DNA-Sequenzen kodierten Proteine und Proteinvarianten, ihre Expression, Gewinnung und Nutzung. Anwendungsgebiete sind die molekularbiologische, medizinische und pharmazeutische

Forschung, die medizinische Diagnostik und Therapie, die pharmazeutische und die biotechnologische Industrie.

Matrix-Metalloproteasen hydrolysieren Proteine der extrazellulären Matrix. Sie verändern die Matrixstruktur und beeinflußen Zell-Matrix-Wechselwirkungen. Zu den Matrix-Metalloproteasen gehören Kollagenasen, Gelatinasen, Stromelysine und Metalloelastasen /1/.

Die Enzyme sind u. a. an folgenden physiologischen Prozessen beteiligt: Ovulation /2 /, Embryoimplantation in den Uterus /3 /, Zellmigrationen und Gewebeumlagerungen während der Embryogenese /4 /, Involution von Brustdrüse /5 / und Uterus /6/, Angiogenese /7/. Matrix-Metalloproteasen spielen eine wichtige Rolle in der Wundheilung und Narbenbildung /8 /, bei der Metastasierung von Tumorzellen /9, 10/, bei rheumatischer Arthritis und bei Osteoarthritis /11, 12/, bei periodontalen Erkrankungen /13/.

Alle Matrix-Metalloproteasen enthalten im aktiven Zentrum ein $Zn^{2+}$ - Ion. Die Aktivierung der in Form inaktiver Proenzyme synthetisierten Matrix-Metalloproteasen erfordert die Lösung einer Bindung zwischen dem $Zn^{2+}$ - Ion im aktiven Zentrum und einem Cys-Rest im N-terminalen Propeptid von Matrix-Metalloproteasen (Cystein-Schalter) /14/. Matrix-Metalloproteasen bestehen aus mehreren Proteindomänen, die Homologie zueinander aufweisen /1, 14/. Während die Protease Matrilysin nur aus einem Propeptid und der Aminosäuresequenz der katalytischen Domäne besteht, enthalten andere Matrix-Metalloproteasen darüberhinaus eine hämopexin-ähnliche Sequenz von ca 200 Aminosäuren. Die Gelatinasen A und B enthalten zusätzliche Aminosäurefolgen. Bekannte Matrix-Metalloproteasen des Menschen, ihre Molekulargewichte und ihre bevorzugten Substrate sind in Tabelle 1 aufgeführt.

Basset et al. (Nature 348: 699, 1990) haben ein Gen entdeckt, welches in Stromazellen aus der Umgebung von invasiven Brusttumoren exprimiert wird. Das Produkt dieses Genes wird als Stromelysin-3 bezeichnet.,

Die verschiedenen Matrix-Metallproteasen zeichnen sich nicht nur durch charakteristische makromolekulare Spezifität für Matrixproteine aus. Ihre Aktivität wird auf verschiedenen molekularen und zellulären Ebenen kontrolliert:

1. Regulation der Synthese von Matrix-Metallproteasen durch Wachstumsfaktoren, Cytokine, Polypeptidhormone, Prostaglandine, Glukocorticoide, Estrogen, Progesteron und andere Effektoren/1,14/.
2. Bindung von Matrix-Metallproteasen an Membranrezeptoren /15/.
3. Aktivierung inaktiver Proenzyme durch spezifische Hydrolyse der jeweiligen Propeptide /16/ oder durch Oxidation /17/.
4. Hemmung von Matrix-Metallproteasen durch spezifische Proteininhibitoren wie TIMP-1, TIMP-2 und TIMP-3 (TIMP = Tissue Inhibitor of Matrix-Metallproteases)/16/.
5. Proteolytischer Abbau von Matrix-Metallproteasen.

Matrix-Metallproteasen werden auf Grund ihrer wichtigen physiologischen Funktionen und ihrer Rolle in der Pathogenese von Krankheiten intensiv untersucht. Es besteht Interesse an der Auffindung und Charakterisierung weiterer Matrix-Metallproteasen.

Die vorliegende Erfindung hat das Ziel, neuartige und bisher nicht bekannte Matrix-Metallproteasen des Menschen für die medizinische Forschung, Diagnostik und Therapie zu erschließen. Die Aufgabe besteht darin, DNA-Sequenzen für Matrix-Metallproteasen zu identifizieren und zu isolieren, sowie die durch die DNA-Sequenzen kodierten Proteine zu charakterisieren.

Die Erfindung wird gemäß den Ansprüchen 1-4 realisiert.

Neuartige Matrix-Metallproteasen werden durch folgende Verfahren ermittelt:

In Sequenzen bekannter Matrix-Metallproteasen werden konservierte Aminosäurefolgen ausgewählt. Zwei geeignete Folgen sind Aminosäuren um einen konservierten Cys-Rest im Propeptid (Cystein-Schalter) und Aminosäuren, die an der $Zn^{2+}$-Bindung im aktiven Zentrum der Enzyme beteiligt sind. Für die ausgewählten Peptide werden degenerierte Oligonukleotide synthetisiert. Mit den Oligonukleotiden und cDNA, welche durch reverse Transkription von mRNA aus Zellen und Geweben erhältlich ist, werden Polymerase-Ketten-Reaktionen (PCR) durchgeführt. Synthetisierte DNA-Fragmente werden kloniert und sequenziert. Die ermittelten Sequenzen werden mit Sequenzen in Gendatenbanken verglichen. PCR-Produkte mit neuartigen, bisher nicht bekannten Nukleotidfolgen und Homologie zu DNA-Sequenzen von Matrix-Metallproteasen werden als Sonden zur Bestimmung der Genexpression und zur Gewinnung vollständiger c-DNA-Sequenzen aus cDNA-Banken genutzt. Die Nukleotidfolgen vollständiger cDNA werden ermittelt. Die Aminosäuresequenzen der zugehörigen Proteine werden durch Translation der kodierenden Nukleotidregionen abgeleitet und nach bekannten Verfahren analysiert.

Folgende cDNA-Sequenzen wurden ermittelt:

1. cDNA-Sequenz mmpm 1a bestehend aus

- 5'-nichttranslatierter Region: Basenpaare 1 bis 141
- kodierender Region: Basenpaare 142 bis 1881
- 3'-nichttranslatierter Region: Basenpaare 1882 bis 3456

(siehe Anlage 1)

2. cDNA-Sequenz mmpm1b bestehend aus

- 5'-nichttranslatierter Region: Basenpaare 1 bis 113
- kodierender Region: Basenpaare 114 bis 1862
- 3'-nichttranslatierter Region: Basenpaare 1863 bis 3437

(siehe Anlage 2)

3. cDNA-Sequenz mmpm2 bestehend aus

- 5'-nichttranslatierter Region: Basenpaare 1 bis 48
- kodierender Region: Basenpaare 49 bis 2058
- 3'-nichttranslatierende Region: Basenpaare 2059 bis 3530

(siehe Anlage 3)

Dabei handelt es sich bei den Sequenzen gemäß Anlagen 1 und 2 (mmpm 1 a und mmpm 1 b) nicht um Ausführungsformen der Erfindung, sondern nur um Beispiele zur Erleichterung des Verständnisses der Erfindung.

Die Erfindung umfaßt auch Varianten dieser Sequenzen sowie homologe DNA-Sequenzen des Menschen und anderer Säugerspezies, die durch Kreuzhybridisierung mit diesen Sequenzen aufgefunden werden. Zur Erfindung gehören ebenfalls Konstrukte, die aus einem Vektor für den Gentransfer in prokaryotische oder eukaryotische Zellen und einer der erfindungsgemäßen Sequenzen bestehen.

Mit Hilfe der DNA-Sequenzen für membrangebundene Matrix-Metalloproteasen können verschiedene Aspekte der Biosynthese kodierter Matrix-Metalloproteasen untersucht werden. Es können die Strukturgene einschließlich flankierender Sequenzen ermittelt werden. cDNA-Sequenzen können als molekulare Sonden zur Analyse der Genexpression in Zellen und Geweben verwendet werden.

Die cDNA-Sequenzen für membrangebundene Matrix-Metalloproteasen kodieren folgende Aminosäuresequenzen:

MMPm1a

```
  1 MTYEMEHLFR CLFAACVSSL VFGSFFNHVV SFSFLFFESL ALSSGVECNG
 51 AISAYCNLCL LGSSDSPASA SQIAGKADAD TMKAMRRPRC GVPDKFGAEI
101 KANVRRKRYA IQGLKWQHNE ITFCIQNYTP KVGEYATYEA IRKAFRVWES
151 ATPLRFREVP YAYIREGHEK QADIMIFFAE GFHGDSTPFD GEGGFLAHAY
201 FPGPNIGGDT HFDSAEPWTV RNEDLNGNDI FLVAVHELGH ALGLEHSSDP
251 SAIMAPFYQW MDTENFVLPD DDRRGIQQLY GGESGFPTKM PPQPRTTSRP
301 SVPDKPKNPT YGPNICDGNF DTVAMLRGEM FVFKERWFWR VRNNQVMDGY
351 PMPIGQFWRG LPASINTAYE RKDGKFVFFK GDKHWVFDEA SLEPGYPKHI
401 KELGRGLPTD KIDAALFWMP NGKTYFFRGN KYYRFNEELR AVDSEYPKNI
451 KVWEGIPESP RGSFMGSDEV FTYFYKGNKY WKFNNQKLKV EPGYPKSALR
501 DWMGCPSGGR PDEGTEEETE VIIIEVDEEG GGAVSAAAVV LPVLLLLLVL
551 AVGLAVFFFR RHGTPRRLLY CQRSLLDKV*
```

MMPm1b

```
  1 MSPAPRPPRC LLLPLLTLGT ALASLGSAQS SSFSPEAWLQ QYGYLPPGDL
 51 RTHTQRSPQS LSAAIAAMQK FYGLQVTGKA DADTMKAMRR PRCGVPDKFG
101 AEIKANVRRK RYAIQGLKWQ HNEITFCIQN YTPKVGEYAT YEAIRKAFRV
151 WESATPLRFR EVPYAYIREG HEKQADIMIF FAEGFHGDST PFDGEGGFLA
201 HAYFPGPNIG GDTHFDSAEP WTVRNEDLNG NDIFLVAVHE LGHALGLEHS
251 SDPSAIMAPF YQWMDTENFV LPDDDRRGIQ QLYGGESGFP TKMPPQPRTT
301 SRPSVPDKPK NPTYGPNICD GNFDTVAMLR GEMFVFKERW FWRVRNNQVM
351 DGYPMPIGQF WRGLPASINT AYERKDGKFV FFKGDKHWVF DEASLEPGYP
401 KHIKELGRGL PTDKIDAALF WMPNGKTYFF RGNKYYRFNE ELRAVDSEYP
451 KNIKVWEGIP ESPRGSFMGS DEVFTYFYKG NKYWKFNNQK LKVEPGYPKS
501 ALRDWMGCPS GGRPDEGTEE ETEVIIIEVD EEGGGAVSAA AVVLPVLLLL
551 LVLAVGLAVF FFRRHGTPRR LLYCQRSLLD KV*
```

MMPm2

```
  1  MGSDPSAPGR PGWTGSLLGD REEAARPRLL PLLLVLLGCL GLGVAAEDAE
 51  VHAENWLRLY GYLPQPSRHM STMRSAQILA SALAEMQRFY GIPVTGVLDE
101  ETKEWMKRPR CGVPDQFGVR VKANLRRRRK RYALTGRKWN NHHLTFSIQN
151  YTEKLGWYHS MEAVRRAFRV WEQATPLVFQ EVPYEDIRLR RQKEADIMVL
201  FASGFHGDSS PFDGTGGFLA HAYFPGPGLG GDTHFDADEP WTFSSTDLHG
251  NNLFLVAVHE LGHALGLEHS SNPNAIMAPF YQWKDVDNFK LPEDDLRGIQ
301  QLYGTPDGQP QPTQPLPTVT PRRPGRPDHR PPRPPQPPPP GGKPERPPKP
351  GPPVQPRATE RPDQYGPNIC DGDFDTVAML RGEMFVFKGR WFWRVRHNRV
401  LDNYPMPIGH FWRGLPGDIS AAYERQDGRF VFFKGDRYWL FREANLEPGY
451  PQPLTSYGLG IPYDRIDTAI WWEPTGHTFF FQEDRYWRFN EETQRGDPGY
501  PKPISVWQGI PASPKGAFLS NDAAYTYFYK GTKYWKFDNE RLRMEPGYPK

551  SILRDFMGCQ EHVEPGPRWP DVARPPFNPH GGAEPGADSA EGDVGDGDGD
601  FGAGVNKDGG SRVVVQMEEV ARTVNVVMVL VPLLLLLCVL GLTYALVQMQ
651  RKGAPRVLLY CKRSLQEWV*
```

Die Erfindung umfaßt auch Varianten dieser Proteine, die durch posttranslationale Proteinmodifizierung, durch proteinchemische Modifikationen oder durch in vitro Mutagenese und Expression von Nukleotidfolgen erhalten werden sowie homologe Proteine des Menschen und anderer Säugerspezies, die durch immunologische Kreuzreaktivität oder vergleichbare Enzymaktivität identifiziert werden. Zur Erfindung gehören ebenfalls Komplexe dieser Proteine mit einem oder mehreren Liganden.

Membrangebundene Matrix-Metalloproteasen 1a, 1b und 2 können aus natürlichen Quellen isoliert werden. Die Proteine können auch durch Gentransfer und Expression in prokaryotischen und eukaryotischen Zellen synthetisiert und aus den rekombinanten Zellen gewonnen werden. Die Verfügbarkeit der Proteine MMPm1a, MMPm1b und MMPm2 ermöglicht Untersuchungen ihrer Struktur und Funktion. Es können Methoden der Bestimmung der enzymatischen Aktivität und Spezifität ausgearbeitet werden. Ausgehend von der Primärstruktur der Proteine MMPm1a, MMPm1b und MMPm2 können monoklonale und polyklonale Antikörper hergestellt werden. Die Antikörper können zur diagnostischen Analytik von MMPm1a, MMPm1b und MMPm2 eingesetzt werden. MMPm1a, MMPm1b und MMPm2 können als Teststrukturen für die Auffindung natürlicher und synthetischer Aktivatoren und Inhibitoren von Matrix-Metalloproteasen verwendet werden.

Zur Charakterisierung von DNA- und Aminosäuresequenzen membrangebundener Matrix-Metalloproteasen werden folgende weitere Angaben gemacht:

Die DNA-Sequenzen für membrangebundene Matrix-Metalloproteasen 1a und 1b unterscheiden sich nur in ihren 5'-nichttranslatierten Regionen und in den unmittelbar darauffolgenden Teilen ihrer kodierenden Regionen. Beginnend mit den Nukleotiden 363 (cDNA für MMPm1a) bzw 344 (cDNA für MMPm1b) sind beide Sequenzen identisch. In der Sequenz für MMPm1a ist ein offener Leserahmen von 580 Codons enthalten. Der Leserahmen beginnt mit den Nukleotiden $A_{142}$TG. Die Umgebung dieser Nukleotide ist für eine effektive Translation jedoch ungünstig. Dagegen befinden sich die im Leserahmen darauffolgenden Nukleotide $A_{154}$TG in einer für einen Translationsstart favorisierten Umgebung. Es ist möglich, daß die Translation des Proteins erst bei dem zweiten ATG beginnt. Die DNA-Sequenz für MMPm1b weist beginnend mit $A_{114}$TG einen offenen Leserahmen von 583 Codons aus. Das Startcodon befindet sich innerhalb der Nukleotidfolge ACCATGT,die eine effektive Translation begünstigt.
Der Translationsstart für MMPm2 befindet sich bei $A_{49}$TG. Der offene Leserahmen der cDNA für MMPm2 enthält 670 Codons.

Die Proteine MMPm1a, MMPm1b und MMPm2 haben berechnete Molekulargewichte von 65 591, 65 900 und 75 813. Die Primärsequenzen von MMPm1a, MMPm1b und MMPm2 sind homolog zu Sequenzen bekannter Matrix-Metalloproteasen. Die drei neuartigen Enzyme enthalten je ein Signalpeptid, eine Prosequenz mit der Cystein-Schalter-Region PRCGVPD und eine Consensus-Sequenz RRKRYA. Es folgen Sequenzen katalytischer Domänen mit der spezifischen Anordnung von drei Histidinresten HELGHALGLEH und hämopexin-homologe Sequenzen. Im Unterschied zu bekannten Matrix-Metalloproteasen enthalten MMPm1a, MMPm1b und MMPm2 darüberhinaus C-terminale Sequenzen mit charakteristischen Folgen hydrophober Aminosäurereste. MMPm1a und MMPm1b weisen die hydrophobe Aminosäurefolge AAAVVLPVLLLLLVLAVGLAV (Aminosäurepositionen 536-556 in MMPm1a, Aminosäurepositionen 539-559 in MMPm1b) auf. Eine analoge Sequenz in MMPm2 ist VVMVLVPLLLLLCVLGLTY (Aminosäurereste 626-645). Die hydrophoben Sequenzen, die noch über die angegebenen Positionen hinausgehen, werden von geladenen Aminosäureresten flankiert. N-terminal überwiegen negativ geladene Glutamin- und Aspartatreste, C-terminal positiv geladenen Arginin- und Lysinreste.
Die Anwesenheit der hydrophoben Sequenzen in MMPm1a, MMPm1b und MMPm2 erlaubt die Schlußfolgerung, daß MMPm1a, MMPm1b und MMPm2 - im Unterschied zu bekannten löslichen Matrix-Metalloproteasen (Tabelle 2) - membran-assoziierte Enzyme sind. Aus den Primärsequenzen folgt, daß Propeptide, katalytische Domänen und hämopexin-homologe Domänen der Proteasen extrazellulär lokalisiert sind. Die äußersten C-Termini der Proteine sollten sich dagegen im Zytosol MMPm1a-, MMPm1b- bzw MMPm2-expremierender Zellen befinden.
Die membrangebundenen Matrix-Metalloproteasen 1a, 1b und 2 enhalten je einen potentiellen Glykosylierungsort.

MMPm1a und MMPm1b unterscheiden sich nur in ihren Signal und Prosequenzen. Die unterschiedliche Struktur der Prosequenzen impliziert unterschiedliche Aktivierungsmechanismen von MMPm1a und MMPm1b. Da die Prosequenzen im Laufe der Aktivierung von Matrix-Metalloproteasen durch Hydrolyse abgetrennt werden, sollte die Aktivierung von MMPm1a und MMPm1b zu einem identischen aktiven Enzym führen.

Northern-Blot-Analysen von mRNA menschlicher Gewebe belegen eine unterschiedliche Expression von MMPm1 und MMPm2. Matrix-Metalloproteasen vom Typ MMPm1 werden vor allem in Lungen-, Plazenta- Nieren-, Ovar-, Prostata-, Dünndarm-, Milz-, Thymus-, und Testisgewebe expremiert. Ihre Expression ist in Herz- und Pankreasgewebe deutlich geringer, in Hirn, Leber und Skelettmuskulatur kaum nachweisbar. MMPm2 wird in Plazenta, Herz, Leber, Skelettmuskel, Niere, Pankreas, Lunge, Testis, Colon und Dünndarm expremiert.

Zusammenfassend wird festgestellt, daß die Erfindung neuartige, bisher nicht bekannte Matrix-Metalloproteasen des Menschen zur Verfügung stellt. Die Kenntnis von cDNA und Proteinsequenzen der Matrix-Metalloproteasen gestattet die weitere Untersuchung von Biosynthese, Struktur und Funktion der Enzyme. Durch Analyse der Gensequenzen können vererbte und erworbene Mutationen aufgefunden werden. Die Bestimmung von Konzentration und Aktivität derMatrix-Metalloproteasen in Zellen, Geweben und Körperausscheidungen ermöglicht diagnostische Aussagen. Die Enzyme können vorteilhaft als Teststrukturen zur Auffindung neuer Pharmaka, darunter Aktivatoren und Inhibitoren von Matrix-Metalloproteasen verwendet werden.

Die Erfindung wird durch folgende Anwendungsbeispiele weiter erläutert, wobei es sich bei den Sequenzen gemäß Anlagen 1 und 2 (mmpm 1 a und mmpm 1 b) nicht um Ausführungsformen der Erfindung, sondern nur um Beispiele zur Erleichterung des Verständnisses der Erfindung handelt.

1. Identifizierung neuartiger DNA-Sequenzen für Matrix-Metallproteasen

Zur Auffindung von cDNA-Sequenzen, die für Matrix-Metallproteasen kodieren, wurde mRNA aus menschlichen Zellen und Tumorgeweben, darunter Neutroblastomzellen, Nierenkarzinom- und Osteosarkomgeweben, isoliert. Die mRNA wurde mit reverser Transkriptase in cDNA umgeschrieben.
In den Proteinsequenzen bekannter Matrix-Metallproteasen wurden zwei konservierte Aminosäurefolgen ausgewählt.

1. Eine Sequenz um einen charakteristischen Cys-Rest in Propeptiden von Matrix-Metallproteasen (Cystein-Schalter):

P-R-C-G-V/N-P-D.

2. Eine Sequenz mit drei His-Resten in der katalytischen Proteindomäne von Matrix-Metallproteasen ($Zn^{2+}$-Bindungsregion):

H - E - L/I/F - G - H - S/V/A - L/M - G

Entsprechend den Aminosäurefolgen wurden degenerierte Oligonukleotid-Primer, die sowohl die Variation der Aminosäuren in den beiden konservierten Sequenzen, als auch die Degeneration des genetischen Codes berücksichtigen, synthetisiert:

1. Propeptid-Primer

```
5' – NN TCT AGA CCC AGI TGT GGI GTI CCI GA – 3'
                            C    AA
```

2. Zn-Bindungsregion-Primer

```
5' – NN GGA TCC CC CAT IGA ATG ICC IAI TTC ATG – 3'
                     G CC G         C   G
```

Beide Primer enthalten je vier Deoxyinosinnukleotide sowie zusätzliche Nukleotide für Erkennungsorte der Restriktionsendonukleasen XbaI (Propeptid-Primer) und BamHI ($Zn^{2+}$-Bindungsregion-Primer).
Die degenerierten Primer wurden zusammen mit cDNA und PCR eingesetzt.

Das Reaktionsgemisch enthielt:

100 ng cDNA
1 µg Propeptid-Primer
1 µg $Zn^{2+}$-Bindungsregion-Primer
2,5 E/100µl DNA Polymerase AmpliTaq
100 µM dNTP
0,01 % Gelatine
50 mM KCl
1,5 mM $MgCl_2$
10 mM Tris-HCl, pH 8,3

Es wurden 30 Reaktionszyklen der Folge 50 sec 94°, 1 min 56°, 2 min 72° durchgeführt.Die amplifizierte DNA wurde mit Phenol/Chloroform extrahiert und anschließend mit den Restriktionsenzymen XbaI und BamHI gespalten. DNA-Fragmente der Größe 350-500 Basenpaare wurden durch Agarose-Gel-Elektrophorese isoliert (Abb. 1) und in dem Plasmid pBluescript SK (Stratagene) kloniert. Einzelne Klone wurden mit T3- und T7-Primern und Sequenase 2.0 (USB/Amersham Life Science) sequenziert. Die erhaltenen Sequenzen wurden mit DNA-Sequenzen in den Datenbanken Genbank und EMBL verglichen. Der Sequenzvergleich erfolgte mit dem Programm FASTA des Programmpaketes HUSAR (GCG Package, Copyright Genetics Computer Group, Inc.). Ausgehend von Nierenkarzinom-cDNA wurden z. B. mehrere hundert Klone mit amplifizierter DNA erhalten, von denen 50 sequenziert wurden. Die Auswertung ergab sowohl bekannte als auch neuartige DNA-Sequenzen. Unter den ersteren waren Sequenzen für menschliche interstitielle Kollagenase und für Matrilysin. Unter den letzteren waren zwei Sequenzen mit Homologie zu menschlichen Matrix-Metalloproteasen. Die zwei neuartigen Sequenzen, die PCRmmpm1 und PCRmmpm2 bezeichnet wurden, enthielten folgende Nukleotide:

<u>PCRmmpm1</u>

TCTAGACCCAGGTGCGGGGTGCCGGACAAGTTTGGGGCTGAGATCAAGGCCAATGTTCGAAGGAAGCGCT
ACGCCATCCAGGGTCTCAAATGGCAACATAATGAAATCACTTTCTGCATCCAGAATTACGCGCCCAAGGT

GGGCGAGTATGCCACATACGAGGCCATTCGCAAGGCGTTCCGCATGTGGGAGAGTGCCACACCACTGCGC
TTGCGCGAGGTGCCCTATGCCTACATCCGTGAGGCCATGAGAAGCAGGCCGACATCATGATCTTCTTTGC
CGAGGGTTCCATGGCGACAGCGCCCTTCGATGGTGAGGGCGGCTTCCTGGCCCGTGCCTACTTCCCAGGC
CCCAACATTGGAGGAGACACCCACTTTGACTCTGCCGAGCCTTGGACTGTCAGGAATGAGGATCTGAATG
GAAATACATCTTCCTGGTGGCTGTGCACGAACTCGGCCACCGCTGGGGGATCC

<u>PCRmmpm2</u>

TCTAGACCCAGGTGCGGGAAGCCGGACCAGTTCGGGGTACGAGTGAAAGCCAACCTGCGGCGGCGTCGGA
AGCGCTACGCCCTCACCGGGAGGAAGTGGAATAACCAACCATCTGACCTTTAGCATCCAGAACTACACCG
GAGAAGTTGGGCTGGTACCACTCGATGGAGGCGGTGCGCAGGGCCTTCCGCGTGTGGGAGCAGGCCACGC
CCCTGGTCTTCCAGGAGGTGCCCTATGAGACATCCGGCTGCGGCGACAGAAGGAGGCCGACATCATGGTA
CTCTTTCCCTCTGGCTTCCACGGCGAACAGCTCGCCGTTTGATGGCACCGGTGGCTTTCTGGCCCACGCC
TATTTCCCTGGCCCCGGCCTAGGCGGGGACACCCATTTTGACGCAGATGAGCCCTGGACCTTCTCCAGCA
CTGACCTGCATGGAAACAACCTCTTCCTGGTGGCAGTGCATGAGCTGGGCCACGCGCTGGGGGATCC

2. Northern-Blot-Analyse

Die Expression von Matrix-Metalloproteasen in menschlichen Geweben wurde mit Hilfe der Fragmente PCRmmpm1 und PCRmmpm2 untersucht. Die Fragmente wurden radioaktiv markiert (Multiprime DNA-Labeling Kit, Amersham Life Science) und mit mRNA auf Nylon (Multiple Tissue Blot, Clontech) hybridisiert. Die Hybridisierungen und anschließenden Waschungen erfolgten unter Standardbedingungen /18/.
Sowohl PCRmmpm1 als auch PCRmmpm2 hybridisieren mit RNA von ca 3,6 kb. Die Experimente belegten jedoch eine unterschiedliche Expression der mit PCRmmpm1 und PCRmmpm2 hybridisierenden mRNA (Abb. 2). Spezifische Transkripte, die mit PCRmmpm1 hybridisieren, sind insbesondere in Lungen-, Plazenta-, Nieren- und Nierenkarzinomgewebe (nicht dargestellt) enthalten. Sie sind in Pankreas- und Herzgewebe in deutlich geringer Menge, in Leber, Skelettmuskulatur und Hirn kaum vertreten.
Messenger RNA, die mit PCRmmpm2 hybridisiert, wird insbesondere in Plazentagewebe synthetisiert. Ihre Expression in Herz, Leber, Skelettmuskel, Niere, Pankreas und Lunge ist jedoch vergleichbar. Sie ist in Hirngewebe deutlich geringer.

3. Isolierung und Sequenzierung von cDNA für membrangebundene Matrix-Metalloproteasen

Eine Lungen-cDNA-Bank im Vektor λgt 11 (Clontech) wurde mittels Phagentransfer auf Nylon und Hybridisierung mit den radioaktiv markierten Sonden PCRmmpm1 und PCRmmpm2 analysiert /18/. Die Hybridisierungen wurden 16 h bei 40° in 50 % Formamid, 5 x SSPE, 5 x Denhardt, 0,5 % SDS, 50 μg/ml denaturierte Heringssperma-DNA durchgeführt. Nach der Hybridisierung wurden die Filter bei Raumtemperatur und bei 65° in 2 x SSC, 0,1 % SDS gewaschen und anschließend in einem Bio-Imaging-Analyzer (BAS 2000, Fuji Photo Film Co,LTD) ausgewertet. Phagenklone, die mit PCRmmpm1 und PCRmmpm2 hybridisierten, wurden anhand spezifisch gebundener Radioaktivität identifiziert. Die aufgefundenen Phagen wurden durch Ausverdünnen schrittweise vereinzelt. Die DNA vereinzelter Phagen wurde isoliert (Quiagen Lambda Kit. Diagen GmbH) und enthaltene cDNA-Inserte wurden in den Plasmidvektor pBluescript SK (Stratagene) eingefügt. Die Inserte wurden anschließend in Teilfragmente zerlegt (Erase-a-Base-System, Promega) und sequenziert (Sequenase 2.0, USB/Amersham Life Science). Ermittelte DNA-Sequenzen wurden mit Hilfe der Programmpakete DNA-STAR (DNA-STAR. Inc) und HUSAR (GCG Package, Copyright Genetics Computer Group, Inc) analysiert. Die Translation der kodierenden Sequenzen und der Vergleich der erhaltenen Aminosäuresequenzen mit bekannten Matrix-Metalloproteasen belegte, daß die neuartigen Sequenzen zu der Familie der Matrix-Metalloproteasen gehören (Abb. 3)

TABELLEN UND ABBILDUNGEN

Tabelle 1:

Matrix-Metalloproteasen des Menschen. Die angegebenen Molekulargewichte sind aus den cDNA-Sequenzen der Enzyme berechnet.

Abb. 1:

Agarose-Gel-Elektrophorese von PCR-Produkten, die mit degenerierten Primern für Matrix-Metalloproteasen und cDNA der menschlichen Neuroblastomzellinie SK-N-SH (Bahn 1), cDNA von Nierenkarzinomgewebe (Bahn 2) und cDNA von Osteosarkomgewebe (Bahn 3) erhalten wurden.

Abb. 2

Northern-Blot-Analyse von mRNA für MMPm1 (oben) und MMPm2 (unten)

Abb. 3

Homologievergleich von MMPm1a, MMPm1b und MMPm2 mit bekannten Matrix-Metalloproteasen des Menschen. Der Vergleich wurde mit dem Programm CLUSTAL durchgeführt. Die Abkürzungen bedeuten: hscollr.pep - Interstitielle Kollagenase, hsclgna.pe - Neutrophile Kollagenase, P08253.swisspro - Gelatinase A, hs4cola.pep - Gelatinase B, hsmmp3a.pep - Stromelysin 1, hsstrom2.pep - Stromelysin 2, hsstrol3.pep - Stromelysin 3.

LITERATUR

1 Matrisian, L.M. (1992) Bioassays 14 455-463
2. Curry, T.E.jr, Mann, J.S., Estes, R.J. und Jones, P.B.C. (1990) Endocrinology 127, 63-68
3. Librach, C.L., Werb, Z., Fitzgerald, M.L., Chiu, K., Corwin, N.M., Esteves, R.A., Grobelny, D., Galardy, R., Damsky, C.H. und Fisher, S.J. (1991) J. Cell Biol. 113, 437-449
4. Brenner, C.A., Adler, R.R., Rappolee, D.A., Pedersen, R.A. und Werb, Z. (1989) Genes Development 3, 848-859
5. Talhouk, R.S., Bissel, M.J. und Werb, Z. (1992) J. Cell Biol. 118, 1271-1282
6. Woessner, J.F. und Taplin, C. (1988) J. Biol. Chem. 263, 16918-16925
7. Folkman, J. und Shing, Y. (1992) J. Biol. Chem. 267, 10931-10934
8. Sakamoto, S. und Sakamoto, M. (1988) Mol. Aspects Med. 10, 301-428
9. Mignatti, P. und Rifkin, D.B. (1993) Physiol. Rev. 73, 161-195
10. Stetler-Stevenson, W.G., Liotta, L.A. und Kleiner, D.E.jr (1993), FASEB J. 7, 1434-1441
11. Dean, D.D., Martel-Pelletier, J., Pelletier, J.-P., Howell, D.S. und Woessner, J.F. jr, (1989) J. Clin. Invest. 84, 678-685
12. McCachren, S.S. (1991) Arthritis Rheum. 34, 1085-1093
13. Birkedahl-Hansen, H. (1993) J. Periodontol. 64, 474-484
14. Woessner, J.F.jr (1991) FASEB J. 5, 2145-2154
15. Monsky, W.L., Kelly, T., Lin, C.-Y., Yeh, Y., Stetler-Stevenson, W.G., Mueller, S.C. und Chen, W.-T. (1993) Cancer Res. 53, 3159- 3164
16. Kleiner, D.E.jr und Stetler-Stevenson, W.G. (1993) Curr. Opinion Cell Biol. 5, 891-897
17. Weiss, S.J., Peppin, G., Ortiz, X., Ragsdale, C. und Test, S.T. (11985) Science 227, 747-749

18. Sambrook, J., Fritsch, E.F. und Maniatis, T. (1989) Molecular Cloning. A Laboratory Manual. 2nd Edition. Cold Spring Harbor Laboratory Press.

| MATRIX-METALLOPROTEASEN | | |
|---|---|---|
| **Protease** | **$M_r$ (kDa)** | **Substrate** |
| Interstitielle Kollagenase (MMP-1) | 54, 1 | Kollagene I, II, III |
| Neutrophile Kollagenase (MMP-5) | 53.4 | Kollagene I, II, III |
| Gelatinase A (MMP-2) | 73,9 | Kollagene IV, V, VII Gelatine, Elastin |
| Gelatinase B (MMP-9) | 78,4 | Kollagene IV, V Gelatine, Elastin |
| Stromelysin 1 (MMP-3) | 54 | Proteoglykane, Fibronektin, Laminin, Gelatine, Kollagene II, IV, V, IX |
| Stromelysin 2 (MMP-10) | 54,1 | Proteoglykane, Fibronektin, Laminin, Gelatine, Kollagene II, IV, V, IX |
| Matrilysin (MMP-7) | 29,7 | Proteoglykane, Fibronektin, Gelatine, Elastin |
| Stromelysin 3 | 54,6 | |
| Metalloelastase | 54 | Fibronektin, Elastin |

```
                             10        20        30        40        50        60
                              .         .         .         .         .         .

MMPm1a.pep         MTYEMEHLFR-----CLFA----ACVSSLV--FGSFFN--------HVVSFS--------
MMPm1b.pep         MS-PAPRPPR-----CLL-------LPLLT--LGTALASLG---SAQSSSFS-PEAWLQQ
MMPm2.pep          MGSDPSAPGRPGWTGSLLGDREEAARPRLLPLLLVLLGCLGLGVAAEDAEVH-AENWLRL
hscollr.pep        M--HSFPPL------LLLLFWGVVS----------HSFPATLETQEQDV--DLVQKYLEK
hsclgna.pep        M--FSLKTL-----PFLLLLHVQIS----------KAFP--VSSKEKNT--KTVQDYLEK
p08253.swisspro    M--EALMARGALTGPLR-ALCLLGCLLSHAAAAPSPIIKFPGDVAPK-TDKELAVQYLNT
hs4cola.pep        M---------SLWQPLVLVLLVLGCCFAAPRQRQSTLVLFPGDLRTNLTDRQLAEEYLYR
hsmmp3a.pep        M-----KSL-----PILLLLCVAVC----------SAYPLDGAARGEDTSMNLVQKYLEN
hsstrom2.pep       M-----MHL-----AFLVLLCLPVC----------SAYPLSGAAKEEDSNKDLAQQYLEK
hsstrol3.pep       MA-------PAAWL------RSAAARALLPPMLLLLLQPPPLLARA--------------
                   *

MMPm1a.pep         FLFFESLALSSGVECNGAISAYCNLCLLGSSDSPASASQIAGKADADTMKAMRRPRCGVP
MMPm1b.pep         YGYLPPGDLRTHTQRSPQ-----SLSAAIAAMQKFYGLQVTGKADADTMKAMRRPRCGVP
MMPm2.pep          YGYLPQPSRHMSTMRSAQI-----LASALAEMQRFYGIPVTGVLDEETKEWMKRPRCGVP
hscollr.pep        Y-YNLKNDGRQVEKRRNSGP----VVEKLKQMQEFFGLKVTGKPDAETLKVMKQPRCGVP
hsclgna.pep        F-YQLPSNQYQSTRKNGTNV----IVEKLKEMQRFFGLNVTGKPNEETLDMMKKPRCGVP
p08253.swisspro    F-YGCPKE------SCNLFV----LKDTLKKMQKFFGLPQTGDLDQNTIETMRKPRCGNP
hs4cola.pep        YGYTRVAEM-----RGESKS----LGPALLLLQKQLSLPETGELDSATLKAMRTPRCGVP
hsmmp3a.pep        Y-YDLEKDVKQFVRRKDSGP----VVKKIREMQKFLGLEVTGKLDSDTLEVMRKPRCGVP
hsstrom2.pep       Y-YNLEKDVKQF-RRKDSNL----IVKKIQGMQKFLGLEVTGKLDTDTLEVMRKPRCGVP
hsstrol3.pep       ---LPPDVHHLHAERRGPQPWHAALPSSPAPAP-------ATQEAPRPASSLRPPRCGVP
                                            +                  ++  +  + + ++ **** *

MMPm1a.pep         DKFGAEIKANV--RRKRYAIQGLKWQHNEITFCIQNYTPKVGEYATYEAIRKAFRVWESA
MMPm1b.pep         DKFGAEIKANV--RRKRYAIQGLKWQHNEITFCIQNYTPKVGEYATYEAIRKAFRVWESA
MMPm2.pep          DQFGVRVKANLRRRRKRYALTGRKWNNHHLTFSIQNYTEKLGWYHSMEAVRRAFRVWEQA
hscollr.pep        DVAQFVLTEGNP-----------RWEQTHLTYRIENYTPDLPRADVDHAIEKAFQLWSNV
hsclgna.pep        DSGGFMLTPGNP-----------KWERTNLTYRIRNYTPQLSEAEVERAIKDAFELWSVA
p08253.swisspro    DVANYNFFPRKP-----------KWDKNQITYRIIGYTPDLDPETVDDAFARAFQVWSDV
hs4cola.pep        DLGRFQTFEGDL-----------KWHHHNITYWIQNYSEDLPRAVIDDAFARAFALWSAV
hsmmp3a.pep        DVGHFRTFPGIP-----------KWRKTHLTYRIVNYTPDLPKDAVDSAVEKALKVWEEV
hsstrom2.pep       DVGHFSSFPGMP-----------KWRKTHLTYRIVNYTPDLPRDAVDSAIEKALKVWEEV
hsstrol3.pep       DPSDG---LSARNRQKRFVLSGGRWEKTDLTYRILRFPWQLVQEQVRQTMAEALKVWSDV
                   *                       +* + ++*+ *  ++ ++        +   *+ +*+ +

MMPm1a.pep         TPLRFREVPYAYIREGHEKQADIMIFFAEGFHGDSTPFDGEGGFLAHAYFPGPNIGGDTH
MMPm1b.pep         TPLRFREVPYAYIREGHEKQADIMIFFAEGFHGDSTPFDGEGGFLAHAYFPGPNIGGDTH
MMPm2.pep          TPLVFQEVPYEDIRLRRQKEADIMVLFASGFHGDSSPFDGTGGFLAHAYFPGPGLGGDTH
hscollr.pep        TPLTFTKV--------SEGQADIMISFVRGDHRDNSPFDGPGGNLAHAFQPGPGIGGDAH
hsclgna.pep        SPLIFTRI--------SQGEADINIAFYQRDHGDNSPFDGPNGILAHAFQPGQGIGGDAH
p08253.swisspro    TPLRFSRI--------HDGEADIMINFGRWEHGDGYPFDGKDGLLAHAFAPGTGVGGDSH
hs4cola.pep        TPLTFTRV--------YSRDADIVIQFGVAEHGDGYPFDGKDGLLAHAFPPGPGIQGDAH
hsmmp3a.pep        TPLTFSRL--------YEGEADIMISFAVREHGDFYPFDGPGNVLAHAYAPGPGINGDAH
hsstrom2.pep       TPLTFSRL--------YEGEADIMISFAVKEHGDFYSFDGPGHSLAHAYPPGPGLYGDIH
hsstrol3.pep       T?LTFTEV--------HEGRADIMIDFARYWDGDDLPFDGPGGILAHAFFPKTHREGDVH
                   +** *  +                *** + *    + *  +*** +  ****+ *    ** *
```

Abb. 3a

```
MMPm1a.pep        FDSAEPWTVRNEDL----------------------------------------------
MMPm1b.pep        FDSAEPWTVRNEDL----------------------------------------------
MMPm2.pep         FDADEPWTFSSTDL----------------------------------------------
hscollr.pep       FDEHERWTNNFT------------------------------------------------
hsclgna.pep       FDAEETWTNTSA------------------------------------------------
p08253.swisspro   FDDDELWTLGEGQVVRVKYGNADGEYCKFPFLFNGKEYNSCTDTGRSDGFLWCSTTYNFE
hs4cola.pep       FDDDELWSLGKGVVVPTRFGNADGAACHFPFIFEGRSYSACTTDGRSDGLPWCSTTANYD
hsmmp3a.pep       FDDDEQWTKDTT------------------------------------------------
hsstrom2.pep      FDDDEKWTEDAS------------------------------------------------
hsstrol3.pep      FDYDETWTIGDDQ-----------------------------------------------
                  **  * *+   +

MMPm1a.pep        ------------------------------------------------------------
MMPm1b.pep        ------------------------------------------------------------
MMPm2.pep         ------------------------------------------------------------
hscollr.pep       ------------------------------------------------------------
hsclgna.pep       ------------------------------------------------------------
p08253.swisspro   KDGKYGFCPHEALFTMGGNAEGQPCKFPFRFQGTSYDSCTTEGRTDGYRWCGTTEDYDRD
hs4cola.pep       TDDRFGFCPSERLYTRDGNADGKPCQFPFIFQGQSYSACTTDGRSDGYRWCATTANYDRD
hsmmp3a.pep       ------------------------------------------------------------
hsstrom2.pep      ------------------------------------------------------------
hsstrol3.pep      ------------------------------------------------------------

MMPm1a.pep        ------------------------------------------------------------
MMPm1b.pep        ------------------------------------------------------------
MMPm2.pep         ------------------------------------------------------------
hscollr.pep       ------------------------------------------------------------
hsclgna.pep       ------------------------------------------------------------
p08253.swisspro   KKYGFCPETAMSTV-GGNSEGAPCVFPFTFLGNKYESCTSAGRSDGKMWCATTANYDDDR
hs4cola.pep       KLFGFCPTRADSTVMGGNSAGELCVFPFTFLGKEYSTCTSEGRGDGRLWCATTSNFDSDK
hsmmp3a.pep       ------------------------------------------------------------
hsstrom2.pep      ------------------------------------------------------------
hsstrol3.pep      ------------------------------------------------------------

MMPm1a.pep        -------NGNDIFLVAVHELGHALGLEHSSDPSAIMAPFYQ-WMDTENFVLPDDDRRGIQ
MMPm1b.pep        -------NGNDIFLVAVHELGHALGLEHSSDPSAIMAPFYQ-WMDTENFVLPDDDRRGIQ
MMPm2.pep         -------HGNNLFLVAVHELGHALGLEHSSNPNAIMAPFYQ-WKDVDNFKLPEDDLRGIQ
hscollr.pep       --------EYNLHRVAAHELGHSLGLSHSTDIGALMYPSY-TF--SGDVQLAQDDIDGIQ
hsclgna.pep       --------NYNLFLVAAHEFGHSLGLAHSSDPGALMYPNY-AFRETSNYSLPQDDIDGIQ
p08253.swisspro   KWGFCPDQGYSLFLVAAHEFGHAMGLEHSQDPGALMAPIYT-YTK--NFRLSQDDIKGIQ
hs4cola.pep       KWGFCPDQGYSLFLVAAHEFGHALGLDHSSVPEALMYPMYR-FTE--GPPLHKDDVNGIR
hsmmp3a.pep       --------GTNLFLVAAHEIGHSLGLFHSANTEALMYPLYHSLTDLTRFRLSQDDINGIQ
hsstrom2.pep      --------GTNLFLVAAHELGHSLGLFHSANTEALMYPLYNSFTELAQFRLSQDDVNGIQ
hsstrol3.pep      --------GTDLLQVAAHEFGHVLGLQHTTAAKALMSAFYT-FRYP--LSLSPDDCRGVQ
                          + ++  **+**+** +** *+    *+* + *          *  **  *++
```

Abb. 3b

```
MMPm1a.pep        QLYGGESG-----------FPTKMPP--QP-RTTSRPSVPDKPKNPT------------
MMPm1b.pep        QLYGGESG-----------FPTKMPP--QP-RTTSRPSVPDKPKNPT------------
MMPm2.pep         QLYGTPDG----QPQPTQPLPTVTPR--RPGRPDHRPPRPPQPPPPGGKPERPPKPGPPV
hscollr.pep       AIYGRSQN--------------------------------PVQ---------PIGPQTPK
hsclgna.pep       AIYGLSSN--------------------------------PIQ---------PTGPSTPK
p08253.swisspro   ELYGASPDIDL-----------------GTGPT-------PTLGPVTP------------
hs4cola.pep       HLYGPRPEPEPRPPTTTTPQPTAPPTVCPTGPPTVHPSERPTAGPTGPPSAGPTGPPTAG
hsmmp3a.pep       SLYGPPPD--------------------------------SPETPLVPTEPVPPEPGTPA
hsstrom2.pep      SLYGPPPA--------------------------------STEEPLVPTKSVPSGSEMPA
hsstrol3.pep      HLYG-------------QPWPTVTSR--TPALG---------PQAGIDTNEIAP-----L
                   +**

MMPm1a.pep        ----------YGPNICDGN--FDTVAMLRGEMFVFKERWFWRVRNNQVMD-GYPMPIGQF
MMPm1b.pep        ----------YGPNICDGN--FDTVAMLRGEMFVFKERWFWRVRNNQVMD-GYPMPIGQF
MMPm2.pep         QPRATERPDQYGPNICDGD--FDTVAMLRGEMFVFKGRWFWRVRHNRVLD-NYPMPIGHF
hscollr.pep       A-------------CDSKLTFDAITTIRGEVMFFKDRFYMR-TNPFYPEVELNF-TSVF
hsclgna.pep       P-------------CDPSLTFDAITTLRGEILFFKDRYFWR-RHPQLQRVEMNF-ISLF
p08253.swisspro   -------------EICKQDIVFDGIAQIRGEIFFFKDRFIWRTVTPRD-KPMGPLLVATF
hs4cola.pep       PSTATTVPLSPVDDACNVNI-FDAIAEIGNQLYLFKDGKYWRFSEGRGSRPQGPFLIADK
hsmmp3a.pep       N-------------CDPALSFDAVSTLRGEILIFKDRHFWR-KSLRKLEPELHL-ISSF
hsstrom2.pep      K-------------CDPALSFDAISTLRGEYLFFKDRYFWR-RSHWNPEPEFHL-ISAF
hsstrol3.pep      EPDAP-------PDACEAS--FDAVSTIRGELFFFKAGFVWRLRGGQLQP-GYPALASRH
                                 *+    **+++ + ++   **+    *               +

MMPm1a.pep        WRGLPASINTAYER-KDGKFVFFKGDKHWVFDEASLEPGYPKHI-KELGRGLPTDKIDAA
MMPm1b.pep        WRGLPASINTAYER-KDGKFVFFKGDKHWVFDEASLEPGYPKHI-KELGRGLPTDKIDAA
MMPm2.pep         WRGLPGDISAAYER-QDGRFVFFKGDRYWLFREANLEPGYPQPL-TSYGLGIPYDRIDTA
hscollr.pep       WPQLPNGLEAAYEFADRDEVRFFKGNKYWAVQGQNVLHGYPKDIYSSFGFPRTVKHIDAA
hsclgna.pep       WPSLPTGIQAAYEDFDRDLIFLFKGNQYWALSGYDILQGYPKDI-SNYGFPSSVQAIDAA
p08253.swisspro   WPELPEKIDAVYEAPQEEKAVFFAGNEYWIYSASTLERGYPKPL-TSLGLPPDVQRVDAA
hs4cola.pep       WPALPRKLDSVFEEPLSKKLFFFSGRQVWVYTGASVL-G-PRRL-DKLGLGADVAQVTGA
hsmmp3a.pep       WPSLPSGVDAAYEVTSKDLVFIFKGNQFWAIRGNEVRAGYPRGIHT-LGFPPTVRKIDAA
hsstrom2.pep      WPSLPSYLDAAYEVNSRDTVFIFKGNEFWAIRGNEVQAGYPRGIHT-LGFPPTIRKIDAA
hsstrol3.pep      WQGLPSPVDAAFED-AQGHIWFFQGAQYWVYDGEKPVLG-PAPL-TELG--LVRFPVHAA
                  *+ **  + +++*        +* *   *   + +   * *  + +  *       + +*

MMPm1a.pep        LFWMPN-GKTYFFRGNKYYRFNEELRAVDSEYPKNIK-VWEGIPESPRGSFMGSDEVFTY
MMPm1b.pep        LFWMPN-GKTYFFRGNKYYRFNEELRAVDSEYPKNIK-VWEGIPESPRGSFMGSDEVFTY
MMPm2.pep         IWWEPT-GHTFFFQEDRYWRFNEETQRGDPGYPKPIS-VWQGIPASPKGAFLSNDAAYTY
hscollr.pep       LS-EENTGKTYFFVANKYWRYDEYKRSMDPGYPKMIAHDFPGIGHKVDAV--FMKDGFFY
hsclgna.pep       VF---YRSKTYFFVNDQFWRYDNQRQFMEPGYPKSISGAFPGIESKVDAV--FQQEHFFH
p08253.swisspro   FN-WSKNKKTYIFAGDKFWRYNEVKKKMDPGFPKLIADAWNAIPDNLDAVVDLQGGGHSY
hs4cola.pep       LR-SGRGKM-LLFSGRRLWRFDVKAQMVDPRSASEVDRMFPGVP--LDTHDVFQYREKAY
hsmmp3a.pep       IS-DKEKNKTYFFVEDKYWRFDEKRNSMEPGFPKQIAEDFPGIDSKIDAV--FEEFGFFY
hsstrom2.pep      VS-DKEKKKTYFFAADKYWRFDENSQSMEQGFPRLIADDFPGVEPKVDAV--LQAFGFFY
hsstrol3.pep      LVWGPEKNKIYFFRGRDYWRFHPSTRRVDSPVPRRAT-DWRGVPSEIDAAFQDADG-YAY
                             +* +   +*++   +  +   ++     + ++     +          +
```

Abb. 3c

```
MMPm1a.pep        FYKGNKYWKFNNQ-KLKVEPGYPKSALRDWMGC----------PSGGRP----DEGTEEE
MMPm1b.pep        FYKGNKYWKFNNQ-KLKVEPGYPKSALRDWMGC----------PSGGRP----DEGTEEE
MMPm2.pep         FYKGTKYWKFDNE-RLRMEPGYPKSILRDFMGCQEHVEPGPRWPDVARPPFNPHGGAEPG
hscoll1r.pep      FFHGTRQYKFDPKT--KRILTL--QKANSWFNCRKNx-----------------------
hsclgna.pep       VFSGPRYYAFDLIA--QRVTRV--ARGNKWLNCRYGx-----------------------
p08253.swisspro   FFKGAYYLKLENQS-LKSV-KFG-SIKSDWLGCx--------------------------
hs4cola.pep       FCQDRFYWRVSSRSELNQVDQVG-YVTYDILQCPEDx-----------------------
hsmmp3a.pep       FFTGSSQLEFDPNA--KKVTHT--LKSNSWLNCx--------------------------
hsstrom2.pep      FFSGSSQFEFDPNA--RMVTHI--LKSNSWLHCx--------------------------
hsstrol3.pep      FLRGRLYWKFDP-VKVKALEGFPRLVGPDFFGCAEPANTFLx--------------------
                     +      +                     *

MMPm1a.pep        TEVIIIEVDEEGGG----------------------AVSAAAVVLPVLLLLLVLAVGLAV
MMPm1b.pep        TEVIIIEVDEEGGG----------------------AVSAAAVVLPVLLLLLVLAVGLAV
MMPm2.pep         ADSAEGDVGDGDGDFGAGVNKDGGSRVVVQMEEVARTVNVVMVLVPLLLLLCVLGLTYAL
hscoll1r.pep      ------------------------------------------------------------
hsclgna.pep       ------------------------------------------------------------
p08253.swisspro   ------------------------------------------------------------
hs4cola.pep       ------------------------------------------------------------
hsmmp3a.pep       ------------------------------------------------------------
hsstrom2.pep      ------------------------------------------------------------
hsstrol3.pep      ------------------------------------------------------------

MMPm1a.pep        FFFRRHGTPRRLLYCQRSLLDKVx
MMPm1b.pep        FFFRRHGTPRRLLYCQRSLLDKVx
MMPm2.pep         VQMQRKGAPRVLLYCKRSLQEWVx
hscoll1r.pep      ------------------------
hsclgna.pep       ------------------------
p08253.swisspro   ------------------------
hs4cola.pep       ------------------------
hsmmp3a.pep       ------------------------
hsstrom2.pep      ------------------------
hsstrol3.pep      ------------------------
```

Abb. 3d

**Anlage 1**

**cDNA-Sequenz MMPm1a**

```
   1 ACCATTTTGC ATTCCCACAG CAGTGAATGA GAGCTCCTGT TTCTCCACAT
  51 TCTCACCAGC ATTTGGTGTT GCTGGTGTTC TGGATTTTGG CCATTCTAAT
 101 AGGTGTGTCA TGGTATCTCA TTGTTTTAAT TTGCATTTCT GATGACATAT
 151 GAGATGGAGC ATCTTTTCAG ATGCTTATTT GCTGCCTGTG TATCTTCTTT
 201 GGTCTTTGGC TCATTTTTTA ATCACGTTGT TTCCTTTTCC TTTCTTTTTT
 251 TTGAGAGTCT TGCTCTGTCA TCCGGGGTGG AGTGCAATGG TGCAATCTCA
 301 GCCTACTGCA ACCTCTGTCT CCTGGGTTCA AGTGATTCTC CTGCCTCAGC
 351 CTCCCAAATA GCTGGCAAAG CTGATGCAGA CACCATGAAG GCCATGAGGC
 401 GCCCCCGATG TGGTGTTCCA GACAAGTTTG GGGCTGAGAT CAAGGCCAAT
 451 GTTCGAAGGA AGCGCTACGC CATCCAGGGT CTCAAATGGC AACATAATGA
 501 AATCACTTTC TGCATCCAGA ATTACACCCC CAAGGTGGGC GAGTATGCCA
 551 CATACGAGGC CATTCGCAAG GCGTTCCGCG TGTGGGAGAG TGCCACACCA
 601 CTGCGCTTCC GCGAGGTGCC CTATGCCTAC ATCCGTGAGG GCCATGAGAA
 651 GCAGGCCGAC ATCATGATCT TCTTTGCCGA GGGCTTCCAT GGCGACAGCA
 701 CGCCCTTCGA TGGTGAGGGC GGCTTCCTGG CCCATGCCTA CTTCCCAGGC
 751 CCCAACATTG GAGGAGACAC CCACTTTGAC TCTGCCGAGC CTTGGACTGT
 801 CAGGAATGAG GATCTGAATG GAAATGACAT CTTCCTGGTG GCTGTGCACG
 851 AGCTGGGCCA TGCCCTGGGG CTCGAGCATT CCAGTGACCC CTCGGCCATC
 901 ATGGCACCCT TTTACCAGTG GATGGACACG GAGAATTTTG TGCTGCCCGA
 951 TGATGACCGC CGGGGCATCC AGCAACTTTA TGGGGGTGAG TCAGGGTTCC
1001 CCACCAAGAT GCCCCCTCAA CCCAGGACTA CCTCCCGGCC TTCTGTTCCT
1051 GATAAACCCA AAAACCCCAC CTATGGGCCC AACATCTGTG ACGGGAACTT
```

1101 TGACACCGTG GCCATGCTCC GAGGGGAGAT GTTTGTCTTC AAGGAGCGCT

1151 GGTTCTGGCG GGTGAGGAAT AACCAAGTGA TGGATGGATA CCCAATGCCC

1201 ATTGGCCAGT TCTGGCGGGG CCTGCCTGCG TCCATCAACA CTGCCTACGA

1251 GAGGAAGGAT GGCAAATTCG TCTTCTTCAA AGGAGACAAG CATTGGGTGT

1301 TTGATGAGGC GTCCCTGGAA CCTGGCTACC CCAAGCACAT TAAGGAGCTG

1351 GGCCGAGGGC TGCCTACCGA CAAGATTGAT GCTGCTCTCT TCTGGATGCC

1401 CAATGGAAAG ACCTACTTCT TCCGTGGAAA CAAGTACTAC CGTTTCAACG

1451 AAGAGCTCAG GGCAGTGGAT AGCGAGTACC CCAAGAACAT CAAAGTCTGG

1501 GAAGGGATCC CTGAGTCTCC CAGAGGGTCA TTCATGGGCA GCGATGAAGT

1551 CTTCACTTAC TTCTACAAGG GGAACAAATA CTGGAAATTC AACAACCAGA

1601 AGCTGAAGGT AGAACCGGGC TACCCCAAGT CAGCCCTGAG GGACTGGATG

1651 GGCTGCCCAT CGGGAGGCCG GCCGGATGAG GGGACTGAGG AGGAGACGGA

1701 GGTGATCATC ATTGAGGTGG ACGAGGAGGG CGGCGGGGCG GTGAGCGCGG

1751 CTGCCGTGGT GCTGCCCGTG CTGCTGCTGC TCCTGGTGCT GGCGGTGGGC

1801 CTTGCAGTCT TCTTCTTCAG ACGCCATGGG ACCCCCAGGC GACTGCTCTA

1851 CTGCCAGCGT TCCCTGCTGG ACAAGGTCTG ACGCCCACCG CCGGCCCGCC

1901 CACTCCTACC ACAAGGACTT TGCCTCTGAA GGCCAGTGGC AGCAGGTGGT

1951 GGTGGGTGGG CTGCTCCCAT CGTCCCGAGC CCCCTCCCCG CAGCCTCCTT

2001 GCTTCTCTCT GTCCCCTGGC TGGCCTCCTT CACCCTGACC GCCTCCCTCC

2051 CTCCTGCCCC GGCATTGCAT CTTCCCTAGA TAGGTCCCCT GAGGGCTGAG

2101 TGGGAGGGCG GCCCTTTCCA GCCTCTGCCC CTCAGGGGAA CCCTGTAGCT

2151 TTGTGTCTGT CCAGCCCCAT CTGAATGTGT TGGGGGCTCT GCACTTGAAG

2201 GCAGGACCCT CAGACCTCGC TGGTAAAGGT CAAATGGGGT CATCTGCTCC

2251 TTTTCCATCC CCTGACATAC CTTAACCTCT GAACTCTGAC CTCAGGAGGC

2301 TCTGGGCACT CCAGCCCTGA AAGCCCCAGG TGTACCCAAT TGGCAGCCTC
2351 TCACTACTCT TTCTGGCTAA AAGGAATCTA ATCTTGTTGA GGGTAGAGAC
2401 CCTGAGACAG TGTGAGGGGG TGGGGACTGC CAAGCCACCC TAAGACCTTG
2451 GGAGGAAAAC TCAGAGAGGG TCTTCGTTGC TCAGTCAGTC AAGTTCCTCG
2501 GAGATCTGCC TCTGCCTCAC CTACCCCAGG GAACTTCCAA GGAAGGAGCC
2551 TGAGCCACTG GGGACTAAGT GGGCAGAAGA AACCCTTGGC AGCCCTGTGC
2601 CTCTCGAATG TTAGCCTTGG ATGGGGCTTT CACAGTTAGA AGAGCTGAAA
2651 CCAGGGGTGC AGCTGTCAGG TAGGGTGGGG CCGGTGGGAG AGGCCCGGGT
2701 CAGAGCCCTG GGGGTGAGCC TGAAGGCCAC AGAGAAAGAA CCTTGCCCAA
2751 ACTCAGGCAG CTGGGGCTGA GGCCCAAAGG CAGAACAGCC AGAGGGGGCA
2801 GGAGGGGACC AAAAAGGAAA ATGAGGACGT GCAGCAGCAT TGGAAGGCTG
2851 GGGCCGGGCA GGCCAGGCCA AGCCAAGCAG GGGGCCACAG GGTGGGCTGT
2901 GGAGCTCTCA GGAAGGGCCC TGAGGAAGGC ACACTTGCTC CTGTTGGTCC
2951 CTGTCCTTGC TGCCCAGGCA GCGTGGAGGG GAAGGGTAGG GCAGCCAGAG
3001 AAAGGAGCAG AGAAGGCACA CAAACGAGGA ATGAGGGGCT TCACGAGAGG
3051 CCACAGGGCC TGGCTGGCCA CGCTGTCCCG GCCTGCTCAC CATCTCAGTG
3101 AGGGGCAGGA GCTGGGGCTC GCTTAGGCTG GGTCCACGCT TCCCTGGTGC
3151 CAGCACCCCT CAAGCCTGTC TCACCAGTGG CCTGCCCTCT CGCTCCCCCA
3201 CCCAGCCCAC CCATTGAAGT CTCCTTGGGC CACCAAAGGT GGTGGCCATG
3251 GTACCGGGGA CTTGGGAGAG TGAGACCCAG TGGAGGGAGC AAGAGGAGAG
3301 GGATGTCGGG GGGGTGGGGC ACGGGGTAGG GGAAATGGGG TGAACGGTGC
3351 TGGCAGTTCG GCTAGATTTC TGTCTTGTTT GTTTTTTTGT TTTGTTTAAT
3401 GTATATTTTT ATTATAATTA TTATATATGA ATTCCAAAAA AAAAAAAAAA
3451 AAAAAA

**Anlage 2**

**cDNA-Sequenz MMPm1b**

1 AAGTTCAGTG CCTACCGAAG ACAAAGGCGC CCCGAGGGAG TGGCGGTGCG
51 ACCCCAGGGC GTGGGCCCGG CCGCGGAGCC CACACTGCCC GGCTGACCCG
101 GTGGTCTCGG ACCATGTCTC CCGCCCCAAG ACCCCCCCGT TGTCTCCTGC
151 TCCCCCTGCT CACGCTCGGC ACCGCGCTCG CCTCCCTCGG CTCGGCCCAA
201 AGCAGCAGCT TCAGCCCCGA AGCCTGGCTA CAGCAATATG GCTACCTGCC
251 TCCCGGGGAC CTACGTACCC ACACACAGCG CTCACCCCAG TCACTCTCAG
301 CGGCCATCGC TGCCATGCAG AAGTTTTACG GCTTGCAAGT AACAGGCAAA
351 GCTGATGCAG ACACCATGAA GGCCATGAGG CGCCCCCGAT GTGGTGTTCC
401 AGACAAGTTT GGGGCTGAGA TCAAGGCCAA TGTTCGAAGG AAGCGCTACG
451 CCATCCAGGG TCTCAAATGG CAACATAATG AAATCACTTT CTGCATCCAG
501 AATTACACCC CCAAGGTGGG CGAGTATGCC ACATACGAGG CCATTCGCAA
551 GGCGTTCCGC GTGTGGGAGA GTGCCACACC ACTGCGCTTC CGCGAGGTGC
601 CCTATGCCTA CATCCGTGAG GGCCATGAGA AGCAGGCCGA CATCATGATC
651 TTCTTTGCCG AGGGCTTCCA TGGCGACAGC ACGCCCTTCG ATGGTGAGGG
701 CGGCTTCCTG GCCCATGCCT ACTTCCCAGG CCCCAACATT GGAGGAGACA
751 CCCACTTTGA CTCTGCCGAG CCTTGGACTG TCAGGAATGA GGATCTGAAT
801 GGAAATGACA TCTTCCTGGT GGCTGTGCAC GAGCTGGGCC ATGCCCTGGG
851 GCTCGAGCAT TCCAGTGACC CCTCGGCCAT CATGGCACCC TTTTACCAGT
901 GGATGGACAC GGAGAATTTT GTGCTGCCCG ATGATGACCG CCGGGGCATC
951 CAGCAACTTT ATGGGGGTGA GTCAGGGTTC CCCACCAAGA TGCCCCCTCA
1001 ACCCAGGACT ACCTCCCGGC CTTCTGTTCC TGATAAACCC AAAAACCCCA
1051 CCTATGGGCC CAACATCTGT GACGGGAACT TTGACACCGT GGCCATGCTC

1101 CGAGGGGAGA TGTTTGTCTT CAAGGAGCGC TGGTTCTGGC GGGTGAGGAA

1151 TAACCAAGTG ATGGATGGAT ACCCAATGCC CATTGGCCAG TTCTGGCGGG

1201 GCCTGCCTGC GTCCATCAAC ACTGCCTACG AGAGGAAGGA TGGCAAATTC

1251 GTCTTCTTCA AAGGAGACAA GCATTGGGTG TTTGATGAGG CGTCCCTGGA

1301 ACCTGGCTAC CCCAAGCACA TTAAGGAGCT GGGCCGAGGG CTGCCTACCG

1351 ACAAGATTGA TGCTGCTCTC TTCTGGATGC CCAATGGAAA GACCTACTTC

1401 TTCCGTGGAA ACAAGTACTA CCGTTTCAAC GAAGAGCTCA GGGCAGTGGA

1451 TAGCGAGTAC CCCAAGAACA TCAAAGTCTG GGAAGGGATC CCTGAGTCTC

1501 CCAGAGGGTC ATTCATGGGC AGCGATGAAG TCTTCACTTA CTTCTACAAG

1551 GGGAACAAAT ACTGGAAATT CAACAACCAG AAGCTGAAGG TAGAACCGGG

1601 CTACCCCAAG TCAGCCCTGA GGGACTGGAT GGGCTGCCCA TCGGGAGGCC

1651 GGCCGGATGA GGGGACTGAG GAGGAGACGG AGGTGATCAT CATTGAGGTG

1701 GACGAGGAGG GCGGCGGGGC GGTGAGCGCG GCTGCCGTGG TGCTGCCCGT

1751 GCTGCTGCTG CTCCTGGTGC TGGCGGTGGG CCTTGCAGTC TTCTTCTTCA

1801 GACGCCATGG GACCCCCAGG CGACTGCTCT ACTGCCAGCG TTCCCTGCTG

1851 GACAAGGTCT GACGCCCACC GCCGGCCCGC CCACTCCTAC CACAAGGACT

1901 TTGCCTCTGA AGGCCAGTGG CAGCAGGTGG TGGTGGGTGG GCTGCTCCCA

1951 TCGTCCCGAG CCCCCTCCCC GCAGCCTCCT TGCTTCTCTC TGTCCCCTGG

2001 CTGGCCTCCT TCACCCTGAC CGCCTCCCTC CCTCCTGCCC CGGCATTGCA

2051 TCTTCCCTAG ATAGGTCCCC TGAGGGCTGA GTGGGAGGGC GGCCCTTTCC

2101 AGCCTCTGCC CCTCAGGGGA ACCCTGTAGC TTTGTGTCTG TCCAGCCCCA

2151 TCTGAATGTG TTGGGGGCTC TGCACTTGAA GGCAGGACCC TCAGACCTCG

2201 CTGGTAAAGG TCAAATGGGG TCATCTGCTC CTTTTCCATC CCCTGACATA

2251 CCTTAACCTC TGAACTCTGA CCTCAGGAGG CTCTGGGCAC TCCAGCCCTG

2301 AAAGCCCCAG GTGTACCCAA TTGGCAGCCT CTCACTACTC TTTCTGGCTA

2351 AAAGGAATCT AATCTTGTTG AGGGTAGAGA CCCTGAGACA GTGTGAGGGG

2401 GTGGGGACTG CCAAGCCACC CTAAGACCTT GGGAGGAAAA CTCAGAGAGG

2451 GTCTTCGTTG CTCAGTCAGT CAAGTTCCTC GGAGATCTGC CTCTGCCTCA

2501 CCTACCCCAG GGAACTTCCA AGGAAGGAGC CTGAGCCACT GGGGACTAAG

2551 TGGGCAGAAG AAACCCTTGG CAGCCCTGTG CCTCTCGAAT GTTAGCCTTG

2601 GATGGGGCTT TCACAGTTAG AAGAGCTGAA ACCAGGGGTG CAGCTGTCAG

2651 GTAGGGTGGG GCCGGTGGGA GAGGCCCGGG TCAGAGCCCT GGGGGTGAGC

2701 CTGAAGGCCA CAGAGAAAGA ACCTTGCCCA AACTCAGGCA GCTGGGGCTG

2751 AGGCCCAAAG GCAGAACAGC CAGAGGGGGC AGGAGGGGAC CAAAAAGGAA

2801 AATGAGGACG TGCAGCAGCA TTGGAAGGCT GGGGCCGGGC AGGCCAGGCC

2851 AAGCCAAGCA GGGGGCCACA GGGTGGGCTG TGGAGCTCTC AGGAAGGGCC

2901 CTGAGGAAGG CACACTTGCT CCTGTTGGTC CCTGTCCTTG CTGCCCAGGC

2951 AGCGTGGAGG GGAAGGGTAG GGCAGCCAGA GAAAGGAGCA GAGAAGGCAC

3001 ACAAACGAGG AATGAGGGGC TTCACGAGAG GCCACAGGGC CTGGCTGGCC

3051 ACGCTGTCCC GGCCTGCTCA CCATCTCAGT GAGGGGCAGG AGCTGGGGCT

3101 CGCTTAGGCT GGGTCCACGC TTCCCTGGTG CCAGCACCCC TCAAGCCTGT

3151 CTCACCAGTG GCCTGCCCTC TCGCTCCCCC ACCCAGCCCA CCCATTGAAG

3201 TCTCCTTGGG CCACCAAAGG TGGTGGCCAT GGTACCGGGG ACTTGGGAGA

3251 GTGAGACCCA GTGGAGGGAG CAAGAGGAGA GGGATGTCGG GGGGGTGGGG

3301 CACGGGGTAG GGGAAATGGG GTGAACGGTG CTGGCAGTTC GGCTAGATTT

3351 CTGTCTTGTT TGTTTTTTTG TTTTGTTTAA TGTATATTTT TATTATAATT

3401 ATTATATATG AATTCCAAAA AAAAAAAAAA AAAAAAA

Anlage 3

**CDNA-Sequenz MMPm2**

```
1    GCGAGGATCC GGCGTGCAGT GTTCCGAGCT GGGCTGGGCG CCGAGAGCAT
51   GGGCAGCGAC CCGAGCGCGC CCGGACGGCC GGGCTGGACG GGCAGCCTCC
101  TCGGCGACCG GGAGGAGGCG GCGCGGCCGC GACTGCTGCC GCTGCTCCTG
151  GTGCTTCTGG GCTGCCTGGG CCTTGGCGTA GCGGCCGAAG ACGCGGAGGT
201  CCATGCCGAG AACTGGCTGC GGCTTTATGG CTACCTGCCT CAGCCCAGCC
251  GCCATATGTC CACCATGCGT TCCGCCCAGA TCTTGGCCTC GGCCCTTGCA
301  GAGATGCAGC GCTTCTACGG GATCCCAGTC ACCGGTGTGC TCGACGAAGA
351  GACCAAGGAG TGGATGAAGC GGCCCCGCTG TGGGGTGCCA GACCAGTTCG
401  GGGTACGAGT GAAAGCCAAC CTGCGGCGGC GTCGGAAGCG CTACGCCCTC
451  ACCGGGAGGA AGTGGAACAA CCACCATCTG ACCTTTAGCA TCCAGAACTA
501  CACGGAGAAG TTGGGCTGGT ACCACTCGAT GGAGGCGGTG CGCAGGGCCT
551  TCCGCGTGTG GGAGCAGGCC ACGCCCCTGG TCTTCCAGGA GGTGCCCTAT
601  GAGGACATCC GGCTGCGGCG ACAGAAGGAG GCCGACATCA TGGTACTCTT
651  TGCCTCTGGC TTCCACGGCG ACAGCTCGCC GTTTGATGGC ACCGGTGGCT
701  TTCTGGCCCA CGCCTATTTC CCTGGCCCCG GCCTAGGCGG GGACACCCAT
751  TTTGACGCAG ATGAGCCCTG GACCTTCTCC AGCACTGACC TGCATGGAAA
801  CAACCTCTTC CTGGTGGCAG TGCATGAGCT GGGCCACGCG CTGGGGCTGG
851  AGCACTCCAG CAACCCCAAT GCCATCATGG CGCCGTTCTA CCAGTGGAAG
901  GACGTTGACA ACTTCAAGCT GCCCGAGGAC GATCTCCGTG GCATCCAGCA
951  GCTCTACGGT ACCCCAGACG GTCAGCCACA GCCTACCCAG CCTCTCCCCA
1001 CTGTGACGCC ACGGCGGCCA GGCCGGCCTG ACCACCGGCC GCCCCGGCCT
1051 CCCCAGCCAC CACCCCCAGG TGGGAAGCCA GAGCGGCCCC CAAAGCCGGG
1101 CCCCCCAGTC CAGCCCCGAG CCACAGAGCG GCCCGACCAG TATGGCCCCA
```

1151 ACATCTGCGA CGGGGACTTT GACACAGTGG CCATGCTTCG CGGGGAGATG
1201 TTCGTGTTCA AGGGCCGCTG GTTCTGGCGA GTCCGGCACA ACCGCGTCCT
1251 GGACAACTAT CCCATGCCCA TCGGGCACTT CTGGCGTGGT CTGCCCGGTG
1301 ACATCAGTGC TGCCTACGAG CGCCAAGACG GTCGTTTTGT CTTTTTCAAA
1351 GGTGACCGCT ACTGGCTCTT TCGAGAAGCG AACCTGGAGC CCGGCTACCC
1401 ACAGCCGCTG ACCAGCTATG GCCTGGGCAT CCCCTATGAC CGCATTGACA
1451 CGGCCATCTG GTGGGAGCCC ACAGGCCACA CCTTCTTCTT CCAAGAGGAC
1501 AGGTACTGGC GCTTCAACGA GGAGACACAG CGTGGAGACC CTGGGTACCC
1551 CAAGCCCATC AGTGTCTGGC AGGGGATCCC TGCCTCCCCT AAAGGGGCCT
1601 TCCTGAGCAA TGACGCAGCC TACACCTACT TCTACAAGGG CACCAAATAC
1651 TGGAAATTCG ACAATGAGCG CCTGCGGATG GAGCCCGGCT ACCCCAAGTC
1701 CATCCTGCGG GACTTCATGG GCTGCCAGGA GCACGTGGAG CCAGGCCCCC
1751 GATGGCCCGA CGTGGCCCGG CCGCCCTTCA ACCCCCACGG GGGTGCAGAG
1801 CCCGGGGCGG ACAGCGCAGA GGGCGACGTG GGGGATGGGG ATGGGGACTT
1851 TGGGGCCGGG GTCAACAAGG ACGGGGGCAG CCGCGTGGTG GTGCAGATGG
1901 AGGAGGTGGC ACGGACGGTG AACGTGGTGA TGGTGCTGGT GCCACTGCTG
1951 CTGCTGCTCT GCGTCCTGGG CCTCACCTAC GCGCTGGTGC AGATGCAGCG
2001 CAAGGGTGCG CCACGTGTCC TGCTTTACTG CAAGCGCTCG CTGCAGGAGT
2051 GGGTCTGACC ACCCAGCGCT CCTGCTAACG GTGCTCAGGG GGCGCCTGTG
2101 GTTCTGAGAT GGCTCCCAGG GGCTCCCTCC GCCCCCAGGT AGGGGCCCCT
2151 CTCAGCCCTC ACACACCCTG TCTGCCCCGC CCTCATTATT TATGTCCAGG
2201 TGTTTGTTTT GTTTTGTTTT TGGCACCTTA CTTGACCATT TGTTTCTGTT
2251 TCCCCGACTG GGCAGGGTG TTTAGAATTT TCTAAATGTA GTTCTGCTCC
2301 AGACAGGGAA TTAGGCCCCC ATCATCCTCT GGCTTGGCCA CAGCCAGGGG

2351 AGCAGAGGGG CAGAGGCCCA CATTGGAAGA GCAGCACCTC CTCAGCCTGA

2401 ACCCCAGGGC TGTAACTGCC AGGCTCTCTT TGCCCAGTTG GAGACTGTCT

2451 GGCCCCCCTG GTCCCCTCCT TCCCAAGTGA GTCTCTCTGG GCCTTAGGAA

2501 GAGCCTTCCA CCCAGGGGCA GCCCCAGGCC AAAGGGGACC TGGAAGGGAG

2551 GTGGGCCGTG GCCCTTGAGT CCCCATTGAG GCTTGGTTCC TTCCCAATCC

2601 AGTGGACTTC GCAGTCCACT TCTGACAGCC TCAGTGACCC TGGCTCCTTG

2651 TGCCAGAGAA CCCAGCCCAC CCCCGGCAGC AGCCCCCAGC TCCCACCTCC

2701 CCTTGGGCCC ACACCTTCTT CCCTCTCTGG AGAAAGGGCC CTGGGCCTGC

2751 CTCACCACGG ACCAAAGGGA GTCTGCCAGG GCCCCTCTCC CCAGGGAAGC

2801 AGCAGCCTCG CCCCTGGCAG AGATGCCTCC CTGAGCTAGA ACCCTCTGTT

2851 CCTTCCCTGT GCCTCCTCCC TCCCTCCCGA CTCACACCAC TAGCCTCAGG

2901 GGTCTGAGCT CCAGCTCCTT TGGGCTTCAG CTGCCAGTGT CCTGAGCCCC

2951 AGGGAGAGGG GGCTGGTGGG TGCCTAGGCC TGGGCAGTGG ATGGCCGTGA

3001 ATGGGTGCCC ACAGTGTCAG GCACTGGGCA TGAGGGGTTC CTCCCCTCCA

3051 GCTCCCTGTG CCCCCAGGGT CCTGGGAGGA GAGACACTGG TGGGGATAGG

3101 CCAGCCGCGC ATCAGACTGT GAACCCCACG AAGGAGCCCA TTGTGGCCTA

3151 AGAGGCTGCC CTCCTGTGCT CAGCCCTGAG GACAGATGCC TCCTTCCTCT

3201 TTTCCTTCCC AAAGCAAGCA AGAGGCCGTG GCTGCTGTGG GAAATGGTAC

3251 TGTACAGCTG GCTCTACTTC CCCATGGCCC TGAGCGAGTG GAGTCTGCCA

3301 CCCAGGATCC CCAAGGCACT TGAGGGGGAA GGATTCTGCT GGCCTCTGCG

3351 AGTGGTTTCT TGTGCACTGG CACCAAGTGC GGGTCCGGCA GCTTCTGCCC

3401 CCTGCAGAAC CGGAGAGCCA GCTAAGGGGT GGGGCTGCGG GGGTTCCGTG

3451 TCCACCCCCA TACATTTATT TCTGTAAATA ATGTGCACTG AATAAATTGT

3501 ACAGCCGGCA AAAAAAAAAA AAAAAAAAAA

## Patentansprüche

**1.** DNA kodierend für eine Matrix-Metalloprotease, **dadurch gekennzeichnet, daß** diese DNA

(a) die Sequenz gemäß der Anlage 3 aufweist oder

(b) in einem Hybridisierungsexperiment unter Verwendung von Nylonfiltern mit einer DNA der Sequenz gemäß Anlage 3 bei 40°C in einer Lösung von 50 % Formamid in 5 x SSPE / 5 x Denhardt-Lösung enthaltend 0,5 % SDS und 50 µg/ml denaturierte Heringssperma-DNA, kreuzhybridisiert, und durch anschließendes Waschen bei Raumtemperatur und bei 65°C in 2 x SSC; 0,1 % SDS, nicht abgewaschen wird.

**2.** Matrix-Metalloprotease kodiert von einer DNA gemäß Anspruch 1.

**3.** Matrix-Metalloprotease gemäß Anspruch 2, wobei die Aminosäuresequenz der Matrix-Metalloprotease durch die Nukleotide 49 bis 2058 der Sequenz gemäß Anlage 3 kodiert wird.

**4.** Matrix-Metalloprotease gemäß Anspruch 3, wobei das Signalpeptid durch posttranslationale Modifizierung entfernt ist.

**5.** Matrix-Metalloprotease gemäß Anspruch 2, 3 oder 4, die am Glykosylierungsort $N_{140}$YT glykolysiert ist.

**6.** Vektor, enthaltend eine DNA gemäß Anspruch 1.

**7.** Vektor gemäß Anspruch 6, ausgewählt aus der Gruppe enthaltend prokaryotische Expressionsvektoren und eukaryotische Expressionsvektoren.

**8.** Zelle, enthaltend einen oder mehrere Vektoren gemäß Anspruch 6 oder 7.

**9.** Zelle gemäß Anspruch 8, ausgewählt aus der Gruppe enthaltend eukaryotische und prokaryotische Zellen.

**10.** Verfahren zur Herstellung von Matrix-Metalloproteasen gemäß der Ansprüche 2 bis 5, wobei die Matrix-Metalloprotease durch Expression einer DNA gemäß Anspruch 1 mittels eines Vektors gemäß Anspruch 6 oder 7 in einer Zelle gemäß Anspruch 8 oder 9 gewonnen wird.

**11.** Verwendung einer Matrix-Metalloprotease gemäß der Ansprüche 2 bis 5 zum Screening von Modulatoren der Matrix-Metalloprotease.

**12.** Verwendung einer Matrix-Metalloprotease gemäß der Ansprüche 2 bis 5 in der molekularbiologischen, medizinischen, pharmazeutischen Forschung, der pharmazeutischen und biotechnologischen Industrie.

**13.** Verwendung einer DNA gemäß Anspruch 1 in der molekularbiologischen, medizinischen, pharmazeutischen Forschung, der pharmazeutischen und biotechnologischen Industrie.

**14.** Monoklonaler oder polyklonaler Antikörper, der spezifisch an eine Matrix-Metalloprotease gemäß der Ansprüche 2 bis 5 bindet.

## Claims

**1.** A DNA encoding a matrix metalloprotease, wherein this DNA

(a) possesses the sequence shown in annex 3, or

(b) cross hybridizes, in a hybridization experiment using nylon filters, with a DNA having the sequence shown in annex 3 at 40°C in a solution of 50% formamide in 5 × SSPE/ 5 × Denhardt's solution containing 0.5% SDS and 50 µg of denatured herring sperm DNA/ml, and is not washed off by subsequent washing at room temperature and at 65°C in 2 × SSC; 0.1% SDS.

**2.** A matrix metalloprotease encoded by a DNA as claimed in claim 1.

**3.** A matrix metalloprotease as claimed in claim 2, where the amino acid sequence of the matrix metalloprotease is encoded by nucleotides 49 to 2058 of the sequence shown in annex 3.

**4.** A matrix metalloprotease as claimed in claim 3, where the signal peptide is removed by post-translational modification.

**5.** A matrix metalloprotease as claimed in claim 2, 3 or 4 which is glycosylated at the glycosylation site $N_{140}YT$.

**6.** A vector containing a DNA as claimed in claim 1.

**7.** A vector as claimed in claim 6, selected from the group containing prokaryotic expression vectors and eukaryotic expression vectors.

**8.** A cell harboring one or more vectors as claimed in claim 6 or 7.

**9.** A cell as claimed in claim 8 selected from the group containing eukaryotic and prokaryotic cells.

**10.** A process for preparing matrix metalloproteases as claimed in claims 2 to 5, which comprises obtaining the matrix metalloprotease by expressing a DNA as claimed in claim 1 using a vector as claimed in claim 6 or 7 in a cell as claimed in claim 8 or 9.

**11.** The use of a matrix metalloprotease as claimed in claims 2 to 5 for screening modulators of the matrix metalloprotease.

**12.** The use of a matrix metalloprotease as claimed in claims 2 to 5 in molecular biological, medical and pharmaceutical research and in the pharmaceutical and biotechnological industries.

**13.** The use of a DNA as claimed in claim 1 in molecular biological, medical and pharmaceutical research and in the pharmaceutical and biotechnological industries.

**14.** A monoclonal or polyclonal antibody which binds specifically to a matrix metalloprotease as claimed in claims 2 to 5.

**Revendications**

**1.** ADN codant pour une métalloprotéase de matrice, **caractérisé en ce que** cet ADN

(a) présente la séquence selon l'Annexe 3 ou
(b) est soumis à une hybridation croisée dans une expérience d'hybridation en utilisant des filtres de nylon avec un ADN de la séquence selon l'Annexe 3 à 40°C dans une solution de formamide à 50 % dans 5 x SSPE/ 5 x solution de Denhardt ayant une teneur de 0,5 % en SDS et de 50 µg/ml en ADN du sperme d'hareng, et il n'est pas éliminé par lavage ultérieur à température ambiante et à 60°C dans 2 x SSC ; 0,1 % SDS.

**2.** Métalloprotéase de matrice codée par un ADN selon la revendication 1.

**3.** Métalloprotéase de matrice selon la revendication 2, où la séquence d'acides aminés de la métalloprotéase de matrice est codée par les nucléotides 49 à 2058 de la séquence selon la revendication 3.

**4.** Métalloprotéase de matrice selon la revendication 3, où le peptide signal est éliminé par modification post-traductionnelle.

**5.** Métalloprotéase de matrice selon la revendication 2, 3 ou 4 qui est glycosylée sur le site de glycosylation $N_{140}YT$.

**6.** Vecteur contenant un ADN selon la revendication 1.

**7.** Vecteur selon la revendication 6, pris dans le groupe comportant des vecteurs d'expression procaryotes et des vecteurs d'expression eucaryotes.

**8.** Cellule contenant un ou plusieurs vecteurs selon la revendication 6 ou 7.

**9.** Cellule selon la revendication 8 prise dans le groupe contenant des cellules eucaryotes et procaryotes.

**10.** Procédé pour la préparation de métalloprotéases de matrice selon la revendication 2 à 5, les métalloprotéases de matrice étant obtenues par expression d'un ADN selon la revendication 1 à l'aide d'un vecteur selon la revendication 6 ou 7 dans une cellule selon la revendication 8 ou 9.

**11.** Utilisation d'une métalloprotéase de matrice selon les revendications 2 à 5 pour la sélection de modulateurs de métalloprotéases de matrice.

**12.** Utilisation d'une métalloprotéase de matrice selon les revendications 2 à 5 dans la recherche de biologie moléculaire, médicale, pharmaceutique, des industries pharmaceutique et biotechnologique.

**13.** Utilisation d'un ADN selon la revendication 1 dans la recherche de biologie moléculaire, médicale, pharmaceutique, des industries pharmaceutique et biotechnologique.

**14.** Anticorps monoclonal ou polyclonal, qui se lie spécifiquement à une métalloprotéase de matrice selon les revendications 2 à 5.

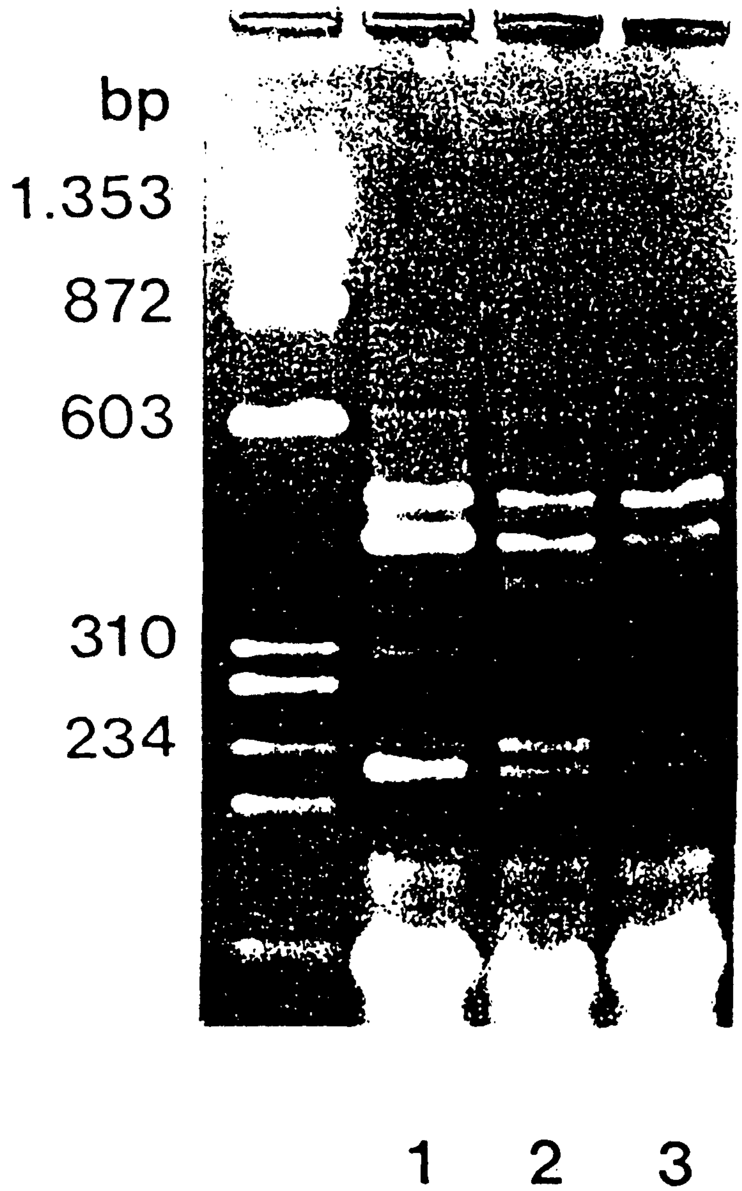
bp
1.353
872
603
310
234
1
2
3

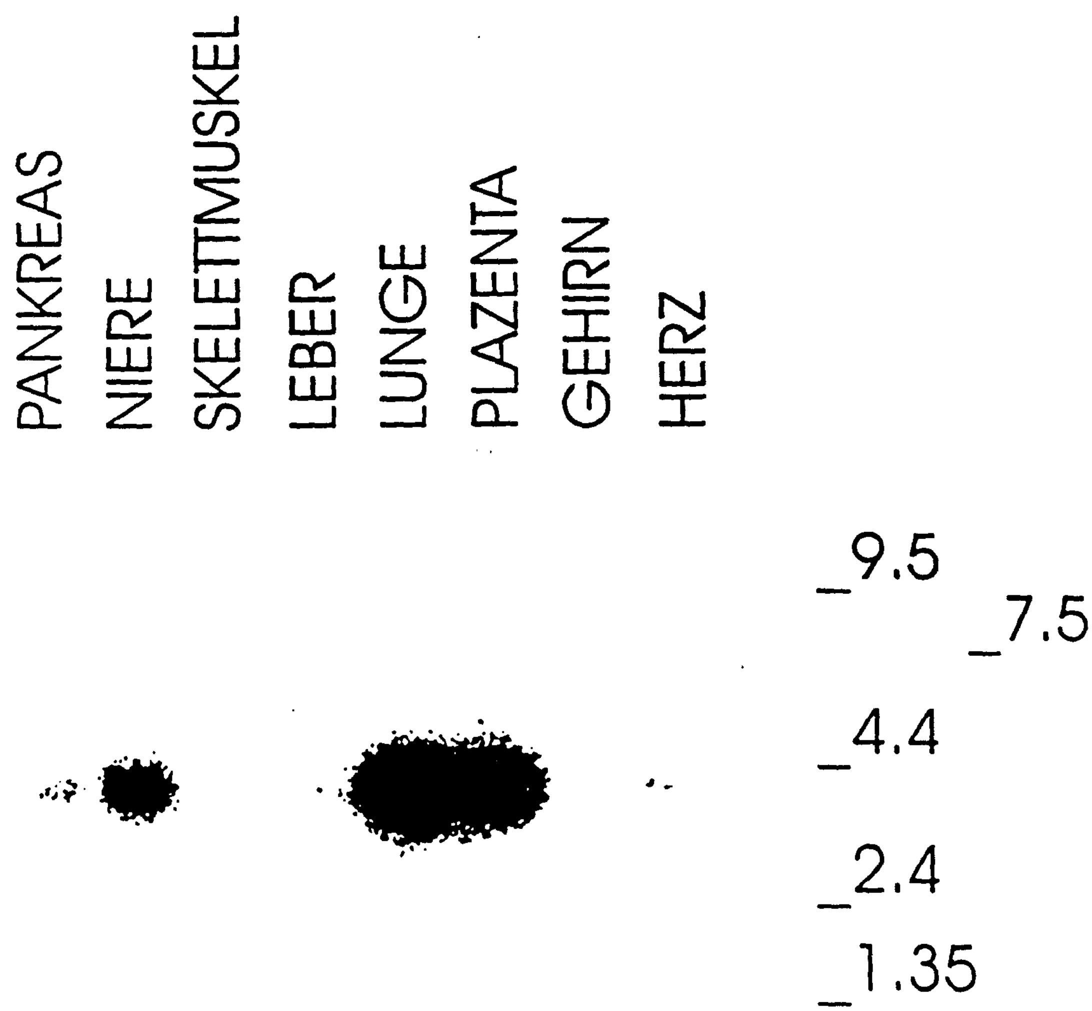
PANKREAS
NIERE
SKELETTMUSKEL
LEBER
LUNGE
PLAZENTA
GEHIRN
HERZ
9.5
7.5
4.4
2.4
1.35

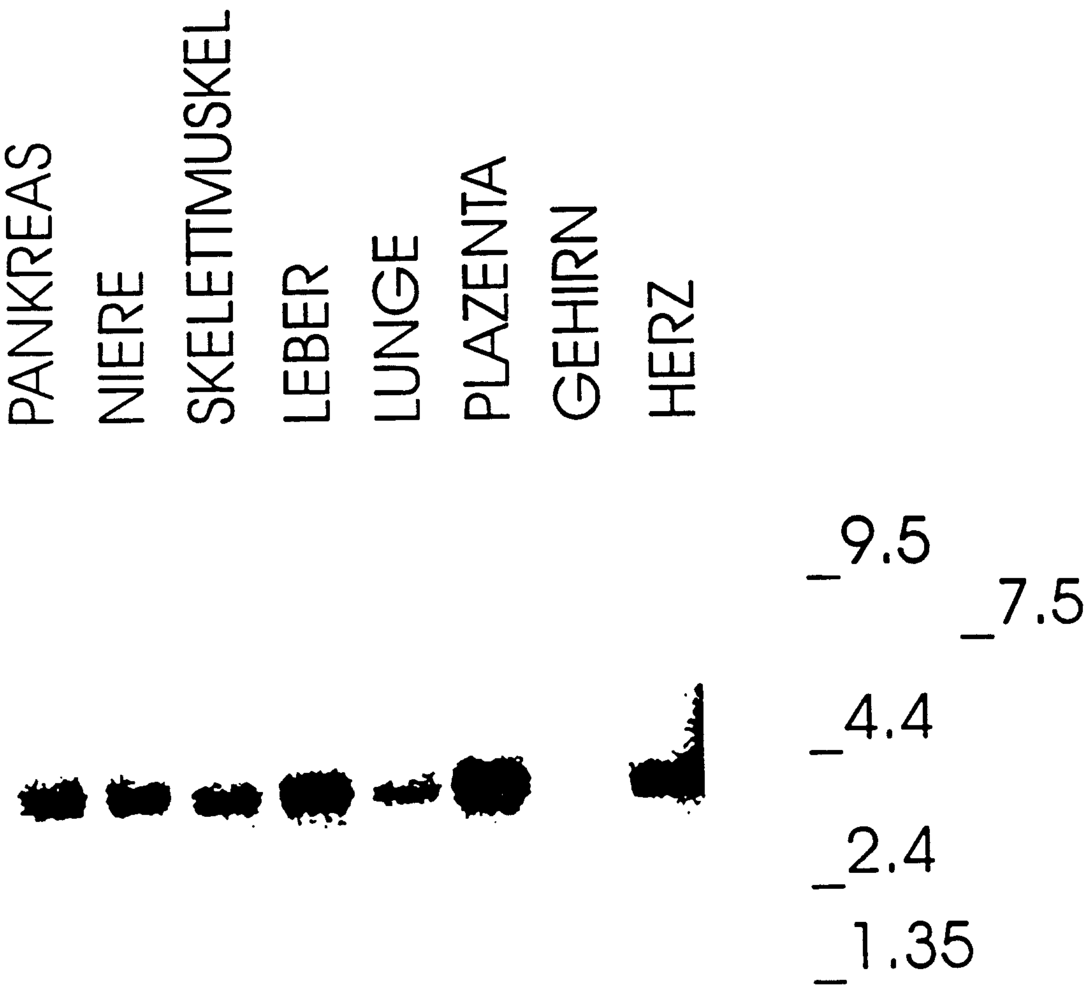
PANKREAS
NIERE
SKELETTMUSKEL
LEBER
LUNGE
PLAZENTA
GEHIRN
HERZ
9.5
7.5
4.4
2.4
1.35